(19) Europäisches Patentamt
European Patent Office
Office européen des brevets

(11) Veröffentlichungsnummer : **0 279 343 B1**

(12) **EUROPÄISCHE PATENTSCHRIFT**

(45) Veröffentlichungstag der Patentschrift : **05.06.91 Patentblatt 91/23**

(21) Anmeldenummer : **88101931.9**

(22) Anmeldetag : **10.02.88**

(51) Int. Cl.[5] : **A61K 31/505**, // (A61K31/505, 31:415), (A61K31/505, 31:155)

# (54) Anthelminthische Wirkstoffkombinationen.

Die Akte enthält technische Angaben, die nach dem Eingang der Anmeldung eingereicht wurden und die nicht in dieser Patentschrift enthalten sind.

(30) Priorität : **19.02.87 DE 3705227**

(43) Veröffentlichungstag der Anmeldung : **24.08.88 Patentblatt 88/34**

(45) Bekanntmachung des Hinweises auf die Patenterteilung : **05.06.91 Patentblatt 91/23**

(84) Benannte Vertragsstaaten : **BE DE ES FR GB IT NL SE**

(56) Entgegenhaltungen :
**FR-A- 2 461 495**
**BIOLOGICAL ABSTRACTS, Nr. 81060383, Philadelphia, US; J. SHIELD: "A study of the effectiveness of mebendazole and pyrantel pamoate as a combination anthelmintic in Papua New-Guinean children", & PAPUA AND NEW GUINEA MEDICAL JOURNAL, 1985, Band 28, Nr. 1, Seiten 41-44**

(56) Entgegenhaltungen :
**BIOLOGICAL ABSTRACTS, Nr. 73052021, Philadelphia, US; M.P.P. PURNOMO et al.: "A comparative study of pyrantel pamoate and a combination of mebendazole and pyrantel pamoate in the treatment of soil transmitted helminths in a rural area indonesia", & SOUTHEAST ASAIAN JOURNAL OF TROPICAL MEDICINE AND PUBLIC HEALTH, 1981. Band 12, Nr. 2, Seiten 236-241**
**BIOLOGICAL ABSTRACTS, Nr. 30047890/RMM, Philadelphia, US; J. NAHMIAS et al.: "Comparative study of four different anthelminthic drugs for necatoriasis", & ISRAEL JOURNAL OF MEDICAL SCIENCES, 1985. Band 21, Nr. 8, Seite 710**
**Purnomo et al. Southeast Asian J. Trop. Med. Publ. Hlth. 12 (1981),236-241,Papua New Guinea Med. J. 28: 41-44,1985**

(73) Patentinhaber : **BAYER AG**
**W-5090 Leverkusen 1 Bayerwerk (DE)**

(72) Erfinder : **Andrews, Peter, Dr.**
**Gellertweg 2**
**W-5600 Wuppertal 1 (DE)**
Erfinder : **Dorn, Hubert, Dr.**
**Pahlkestrasse 71**
**W-5600 Wuppertal 1 (DE)**
Erfinder : **Voege, Herbert, Dr.**
**Martin-Buber-Strasse 41**
**W-5090 Leverkusen 1 (DE)**

EP 0 279 343 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft anthelminthische Wirkstoffkombinationen aus Phenylguanidinen oder Benzimidazolen und Tetrahydropyrimidinen.

Es ist bekannt, daß Phenylguanidine und Benzimidazole anthelminthisch wirksam sind z. B. gegen Ascariden, Hakenwürmer und Peitschenwürmer. Ihre Wirkung gegen Hakenwürmer ist dabei nicht immer voll befriedigend (US-P 3 996 368, 3 993 682).

Es ist bekannt, daß Tetrahydropyrimidine anthelminthisch wirksam sind z. B. gegen Ascariden und Hakenwürmer. Aber auch bei diesen Verbindungen ist die Wirkung gegen Hakenwürmer nicht immer voll befriedigend (US-P 3 502 661).

Es ist bekannt, daß eine Kombination aus Praziquantel (einem Hexahydropyrazinoisochinolin) und Pyrantel (einem Tetrahydropyrimidin) zur Bekämpfung von Necator americanus, einem Dünndarmparasiten des Menschen, eingesetzt werden kann (Isr. J. Med. Sci 21 (1985) S. 710). Über eine Anwendung dieser Mischung in der Veterinärmedizin ist nichts bekannt.

Es wurde gefunden, daß eine Kombination von Phenylguanidinen der Formel I

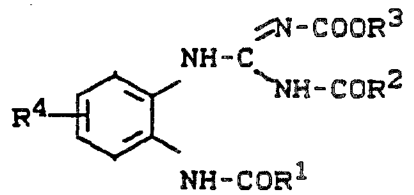

(I)

in welcher
$R^1$ für Wasserstoff, gegebenenfalls substituiertes Alkyl, Cycloalkyl, Alkoxy, Aryl oder Amino steht,
$R^2$ für Wasserstoff, gegebenenfalls substituiertes Alkyl, Cycloalkyl, Alkenyl, Alkinyl, Aryl, Alkoxy, Alkenoxy, steht,
$R^3$ für Wasserstoff, gegebenenfalls substituiertes Alkyl, Cycloalkyl, Alkenyl, Alkinyl, Aryl, steht,
$R^4$ für Alkyl, Alkoxy, Phenoxy, Alkylthio, Phenylthio, Phenylsulfinyl, Phenylsulfonyl, Benzoyl steht, die gegebenenfalls substituiert sind durch Halogen, Alkoxy, Alkylthio, Halogenalkoxy, Halogenalkylthio,
oder Benzimidazolen der Formel II

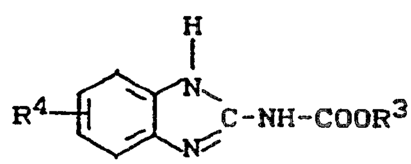

(II)

in welcher
$R^3$ und $R^4$ die bei den Verbindungen der Formel I angegebene Bedeutung haben
zusammen mit Tetrahydropyrimidinen der Formel III

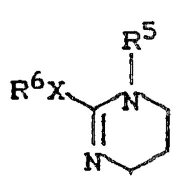

(III)

in welcher
$R^5$ für Wasserstoff oder Alkyl steht,
$R^6$ für gegebenenfalls substituiertes Phenyl oder Thienyl steht,
X für $-(CH_2)_{2-3}-$, $-CH=CH-$ steht
zur Bekämpfung von Hakenwürmern besonders geeignet ist; ausgenommen sind Mischungen von Pyrantel und Mebendazol.

Die Wirkung dieser Kombination geht dabei über die Summe der Wirkung der Einzelwirkstoffe hinaus. Die Wirkstoffe beeinflussen sich in der Kombination synergistisch.

Zu den Phenylguanidinen gehören z.B. Febantel, common name für [[2-[(Methoxyacetyl)amino]-4-(phenylthio)phenyl]carbonimidoyl]biscarbaminsäure dimethylester ; Netobimin, common name für die Verbindung der Formel

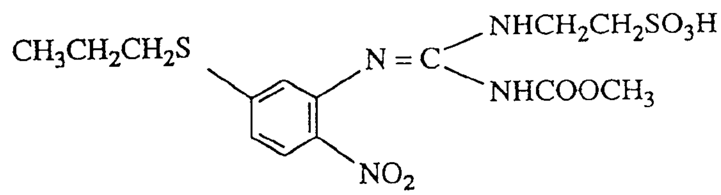

Zu den Benzimidazolen gehören z.B. Fenbendazol, common name für [5-Phenylthio)-1H-benzimidazol-2-yl]carbaminsäure methylester ;
Albendazol, common name für [5(Propylthio)-1H-benzimidazol-2-yl]carbaminsäure methylester ;
Oxibendazol, common name für (5-Propoxy-1H-benzimidazol-2-yl)carbaminsäure methylester ;
Oxfendazol, common name für [5-(Phenylsulfinyl)-1H-benzimidazol-2-yl]carbaminsäure methylester ;
Mebendazol, common name für (5-Benzoyl-1H-benzimidazol-2-yl)-carbaminsäure methylester ;
Flubendazol, common name für [5-(4-Fluorbenzoyl)-1H-benzimidazol-2-yl]carbaminsäure methylester ;
Parbendazol, common name für (5-Butyl-1H-benzimidazol-2-yl)carbaminsäure methylester ;
Luxabendazol, common name für [5-(4-Fluorphenylsulfonyloxy)-1H-benzimidazol-2-yl]carbaminsäure methylester.

Zu den Tetrahydropyrimidinen gehören z.B.
Pyrantel, common name für 1,4,5,6-Tetrahydro-1-methyl-2-[2-(2-thienyl)ethenyl]pyrimidin) ;
Morantel, common name für 1,4,5,6-Tetrahydro-1-methyl-1-methyl-2-[2-(3-methyl-2-thienyl)ethenyl]pyrimidin;
Oxantel, common name für (3-[2-(1,4,5,6-Tetrahydro-1-methyl-2-pyrimidinyl)ethenyl]phenol).

Besonders bevorzugt ist eine Mischung aus Febantel und Pyrantel.

Die erfindungsgemäßen Kombinationen können noch andere Wirkstoffe z. B. gegen Endoparasiten enthalten. Als solche seien genannt : Anthelminthika aus der Klasse der Hexahydropyrazinoisochinoline wie z. B. Praziquantel oder Benzazepine wie z.B. 2-Cyclohexylcarbonyl-1,2,3,4,6,7,8,12b-octahydropyrazino(2,1a)(2)benzazepin4-on, oder Salizylamide wie z. B. Niclosamid.

Die Wirkstoffe sind in der Kombination in folgendem Verhältnis vorhanden :

Phenylguanidin bzw. Benzimidazol zu Tetrahydropyrimidin wie 1 zu 1 bis 2 zu 1.

Die Zubereitungen eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere :

Aus der Ordnung der Pseudophyllidea z.B. : Diphyllobothrium spp., Spirometra spp., Schistocephalus spp., Ligula spp., Bothridium spp., Diplogonoporus spp..

Aus der Ordnung der Cyclophyllidea z.B. : Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B. : Gyrodactylus spp., Dactylogyrus spp., Polystoma spp..

Aus der Unterklasse der Digenea z.B. : Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp.

Metorchis spp., Heterophyes spp., Metagonimus spp..

Aus der Ordnung der Enoplida z.B. : Trichuris spp., Capillaria spp., Trichomosoides spp., Trichinella spp..

Aus der Ordnung der Rhabditia z.B. : Micronema spp., Strongyloides spp..

Aus der Ordnung der Strongylida z.B. : Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostergagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B. : Oxyuris spp., Enterobius spp., Passalurus spp., Syphacia spp., Aspiculuris spp., Heterakis spp..

Aus der Ordnung der Ascaridia z.B. : Ascaris spp., Toxascaris spp., Toxocara spp., Parascaris spp., Anisakis spp., Ascaridia spp..

Aus der Ordnung der Spirurida z.B. : Gnathostoma spp., Physaloptera spp., Thelazia spp., Gongylonema spp., Habronema spp., Parabronema spp., Draschia spp., Dracunculus spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien.

Zu Labor- und Versuchstieren gehören Mäuse, Ratten, Meerschweinchen, Goldhamster, Hunde und Katzen.

Zu den Hobbytieren gehören Hunde und Katzen.

Besonders bevorzugt erfolgt die Anwendung der erfindungsgemäßen Kombination bei Hunden und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser.

Geeignete Zubereitungen sind :

Orale Lösungen, Konzentrate zur oralen Verabreichung nach Verdünnung ;

Emulsionen und Suspensionen zur oralen Anwendung ; Halbfeste Zubereitungen ;

Formulierungen bei, denen der Wirkstoff in einer Salbengrundlage oder in einer Öl in Wasser oder Wasser in Öl Emulsionsgrundlage verarbeitet ist ;

Feste Zubereitungen wie Pulver, Premixe oder Konzentrate, Granulate, Pellets, Tabletten, Boli, Kapseln.

Orale Lösungen werden hergestellt, indem der Wirkstoff in einem geeigneten Lösunsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt : Physiologisch verträgliche Lösungsmittel wie Wasser, Alkohole wie Ethanol, Butanol, Benzylalkohol Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen lösen.

Als Lösungsvermittler seien genannt : Lösungsmittel, die die Lösung des Wirkstoffs im Hauplösungsmittel fördern oder Substanzen die sein Ausfallen verhindern. Beispiele sind Polyvinylpyrrolidon, polyoxyethyliertes Rhizinusöl, polyoxyethylierte Sorbitanester.

Konservierungsmittel sind : Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet.

Emulsionen können oral angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt : Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-N-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt :

Wasser, Alkohole wie z.B. Propylenglycol, Glycerin, Sorbitol und ihre Gemische.

Als Emulgatoren seien genannt : nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether ;

ampholytische Tenside wie Di-Na-N-lauryl-β-iminodiproprionat oder Lecithin ;

anionaktive Tenside, wie Na-Laurylsulfat, Fettalkoholethersulfate, Mono/Dialkylpolyglykoletherorthophosphorsäureester-monoethanolaminsalz ;

kationaktive Tenside wie Cetyltrimethylammoniumchlorid.

Als weitere Hilfsstoffe seien genannt : Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festern Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungsund Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikdrokristalline Cellulose.

Besonders bevorzugt erfolgt die Anwendung der erfindungsgemäßen Kombination in Form von Tabletten.

Anwendungsfertige Zubereitungen enthalten den Wirkstoff in Konzentrationen von 10 ppm-20 Gewichtsprozent, bevorzugt von 0,1-10 Gewichtsprozent.

Zubereitungen die vor Anwendung verdünnt werden, enthalten den Wirkstoff in Konzentrationen von 0,5-90 Gewichtsprozent, bevorzugt von 5 bis 50 Gewichtsprozent.

Die bevorzugte Anwendungskonzentration der erfindungsgemäßen Mischung liegt bei Hund und Katze pro kg Lebendgewicht bei 1-300 mg Tetrahydropyrimidin und 5-50 mg Phenylguanidin bzw. Benzimidazol.

Besonders bevorzugt werden Konzentrationen pro kg von 10-100 mg Tetrahydropyrimidin und 10-30 mg

Phenylguanidin bzw. Benzimidazol eingesetzt.

Ganz besonders bevorzugt wird Tetrahydropyrimidin pro kg mit ca. 15 mg und Phenylguanidin bzw. Benzimidazol mit ca. 15 mg oder ca. 25 eingesetzt.

Weitere anthelminthische Wirkstoffe wie z. B. Praziquantel können der Kombination zur Anwendung in einer Aufwandmenge pro kg von 0,1-20 mg, bevorzugt 1-10 mg, besonders bevorzugt ca. 5 mg, zugesetzt werden.

Beispiel A

Wirkung gegen Ancylostoma caninum beim Hund

Experimentell mit Ancylostoma caninum infizierte Hunde wurden nach Ablauf der Präpatenzzeit des Parasiten behandelt. Die Wirkstoffe wurden als reiner Wirkstoff in Gelatinekapseln oral appliziert.

Der Wirkungsgrad wurde dadurch bestimmt, daß man die mit dem Kot ausgeschiedenen Würmer zählt. Anschließend wird die Anzahl der durch die Behandlung nicht abgetöteten und ausgeschiedenen Würmer nach Sektion der Hunde bestimmt. Dabei wurden folgende Werte erhalten :

| Wirkstoff | Dosis mg/kg | Reduktion der Parasiten in % |
|---|---|---|
| Febantel | 10 | 8 |
| Febantel | 25 | 40 |
| Pyrantel | 15 | 75 |
| Praziquantel | 5 | 0 |
| Febantel | 15 | 93 |
| Pyrantel | 15 | |
| Praziquantel | 5 | |
| Febantel | 25 | 95 |
| Pyrantel | 15 | |
| Praziquantel | 5 | |

Beispiel B

Wirkung gegen Toxocara canis beim Hund

Experimentell mit Toxocara canis infizierte Hunde wurden nach Ablauf der Präpatenzzeit des Parasiten behandelt. Die Wirkstoffe wurden als reiner Wirkstoff in Gelatinekapseln oral appliziert.

Der Wirkungsgrad wurde dadurch bestimmt, daß man die mit dem Kot ausgeschiedenen Würmer zählt. Anschließend wird die Anzahl der durch die Behandlung nicht abgetöteten uno ausgeschiedenen Würmer nach Sektion der Hunde bestimmt. Dabei wurden folgende Werte erhalten :

| Wirkstoff | Dosis mg/kg | Reduktion der Parasiten in % |
|---|---|---|
| Febantel | 10 | 47 |
| Febantel | 25 | 84 |
| Pyrantel | 15 | 75 |
| Praziquantel | 5 | 0 |
| Febantel | 15 | |
| Pyrantel | 15 | 94 |
| Praziquantel | 5 | |
| Febantel | 25 | |
| Pyrantel | 15 | 98 |
| Praziquantel | 5 | |

Beispiel 1

Zusammensetzung :

| | |
|---|---|
| Praziquantel Wirkstoff | 50,0 mg |
| Pyrantel Wirkstoff | 150,0 mg |
| Febantel Wirkstoff | 150,0 mg |
| Milchzucker | 100,0 mg |
| Maisstärke | 143,0 mg |
| Natriumlaurysulfat | 2,0 mg |
| Polyvinylpyrrolidon | 18,0 mg |
| mirkokristalline Cellulose | 49,0 mg |
| kolloidale Keiselsäure | 1,0 mg |
| Magnesiumstearat | 3,0 mg |

Herstellung

Im obigen Verhältnis werden die Wirkstoffe mit Milchzucker und einem Teil der Maisstärke gemischt und mit einer Lösung von Polyvinylpyrrolidon und Natriumlaurylsulfat in Wasser granuliert und das Granulat getrocknet. Das trockene Granulat wird mit den übrigen Hilfsstoffen gemischt. Die preßfertige Mischung wird zu Tabletten mit 660 mg Gewicht verpreßt z. B. auf eine Tablettengröße mit einem Durchmesser von 13 mm.

Beispiel 2

Zusammensetzung :

| | |
|---|---|
| Pyrantel | 50 mg |
| Febantel | 250 mg |
| kolloidale Kieselsäure | 10 mg |
| mikrokristalline Cellulose | 600 mg |
| Vollmilchpulver | 2446 mg |

Herstellung :

Die Substanzen werden in entsprechender Menge im obigen Verhältnis abgewogen und gemischt. Die Mischung wird gesiebt und kann direkt zu weichen Komprimaten von 3,5 g auf einer Tablettenmaschine ver-

preßt werden.

Beispiel 3

Premix zum Mischen unter das Futter

Zusammensetzung :

| | |
|---|---|
| Praziquantel | 1,43 g |
| Febantel | 7,15 g |
| Pyrantelembonat | 4,12 g |
| Weizennachmehl | 87,30 g |

Herstellung :

Die Substanzen werden abgewogen und gemischt. Davon wird je nach Art und Gewicht der Tiere ein entsprechender Teil dem Futter beigemischt-z. B. für einen Hund von 10 kg bei einer Menge von 3,5 g für eine einzige Behandlung.

Beispiel 4

Paste zur oralen Applikation oder zum Mischen unter das Futter

Zusammensetzung :

| | |
|---|---|
| Praziquantel | 5,0 g |
| Pyrantelpamoat | 14,4 g |
| Febantel | 15,0 g |
| Natriumalginat | 1,0 g |
| Polysorbat80 | 0,5 g |
| Methyl-4-hydroxybenzoat | 0,2 g |
| Wasser entmineralisiert | 63,9 g |

Herstellung :

Methyl-4-hydroxybenzoat wird in heißem Wasser gelöst. Nach Erkalten wird Natriumalginat darin aufgelöst, wodurch sich ein Gel bildet. Nach Zufügen von Polysorbat 80 werden die Wirkstoffe darin suspendiert und homogenisiert. Es entsteht eine Paste, die sowohl direkt oral als auch über das Futter appliziert werden kann.

## Ansprüche

### Patentansprüche für die Vertragsstaaten BE, DE, FR, GB, IT, NL, SE

1. Anthelminthisch wirksame Mittel, gekennzeichnet durch einen Gehalt einer Kombination von Phenylguanidinen der Formel I

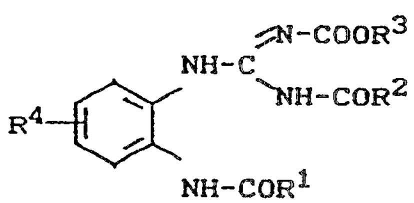

(I)

in welcher
$R^1$ für Wasserstoff, gegebenenfalls substituiertes Alkyl, Cycloalkyl, Alkoxy, Aryl oder Amino steht,

$R^2$ für Wasserstoff, gegebenenfalls substituiertes Alkyl, Cycloalkyl, Alkenyl, Alkinyl, Aryl, Alkoxy, Alkenoxy, steht,
$R^3$ für Wasserstoff, gegebenenfalls substituiertes Alkyl, Cycloalkyl, Alkenyl, Alkinyl, Aryl, Halogenalkoxy, Halogenalkylthio, steht,
$R^4$ für Alkyl, Alkoxy, Phenoxy, Alkylthio, Phenylthio, Phenylsulfinyl, Phenylsulfonyl, Benzoyl steht, die gegebenenfalls substituiert sind durch Halogen, Alkoxy, Alkylthio, Halogenalkoxy, Halogenalkylthio,
oder Benzimidazolen der Formel II

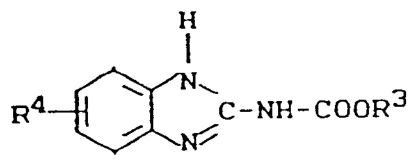 (II)

in welcher
$R^3$ und $R^4$ die bei den Verbindungen der Formel I angegebene Bedeutung haben
zusammen mit Tetrahydropyrimidinen der Formel III

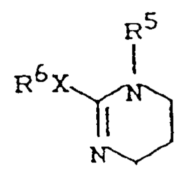 (III)

in welcher
$R^5$ für Wasserstoff oder Alkyl steht,
$R^6$ für gegebenenfalls substituiertes Phenyl oder Thienyl steht,
X für $-(CH_2)_{2-3}-$, $-CH=CH-$ steht
sowie gegebenenfalls weiteren Wirkstoffen, ausgenommen die Mischung zwischen Mebendazol und Pyrantel bzw. Pyrantel-Pamoat.

2. Mittel gemäß Anspruch 1 zur Bekämpfung von Hakenwürmern und Spulwürmern.

3. Verwendung von Mischungen aus Phenylguanidinen der Formel I

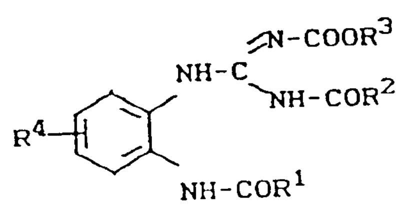 (I)

in welcher
$R^1$ für Wasserstoff, gegebenenfalls substituiertes Alkyl, Cycloalkyl, Alkoxy, Aryl oder Amino steht,
$R^2$ für Wasserstoff, gegebenenfalls substituiertes Alkyl, Cycloalkyl, Alkenyl, Alkinyl, Aryl, Alkoxy, Alkenoxy, steht,
$R^3$ für Wasserstoff, gegebenenfalls substituiertes Alkyl, Cycloalkyl, Alkenyl, Alkinyl, Aryl, steht,
$R^4$ für Alkyl, Alkoxy, Phenoxy, Alkylthio, Phenylthio, Phenylsulfinyl, Phenylsulfonyl, Benzoyl steht, die gegebenenfalls substituiert sind durch Halogen, Alkoxy, Alkylthio, Halogenalkoxy, Halogenalkylthio,
oder Benzimidazolen der Formel II

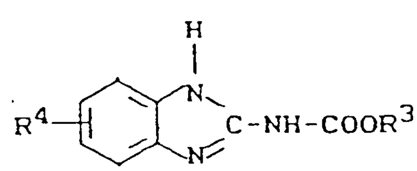 (II)

in welcher
$R^3$ und $R^4$ die bei den Verbindungen der Formel I angegebene Bedeutung haben
mit Tetrahydropyrimidinen der Formel III

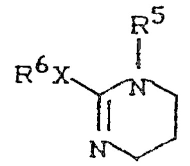

(III)

in welcher
$R^5$ für Wasserstoff oder Alkyl steht,
$R^6$ für gegebenenfalls substituiertes Phenyl oder Thienyl steht,
X für $-(CH_2)_{2-3}-$, $-CH=CH-$ steht,
und gegebenenfalls weiteren Wirkstoffen ausgenommen die Mischung zwischen Mebendazol und Pyrantel bzw. Pyrantel-Pamoat zur Herstellung von anthelmintischen Mitteln.

4. Verwendung von Phenylguanidinen der Formel I

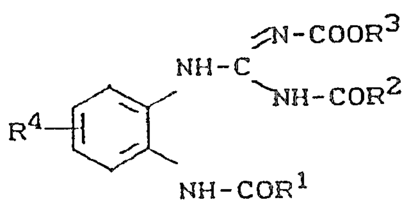

(I)

in welcher
$R^1$ für Wasserstoff, gegebenenfalls substituiertes Alkyl, Cycloalkyl, Alkoxy, Aryl oder Amino steht,
$R^2$ für Wasserstoff, gegebenenfalls substituiertes Alkyl, Cycloalkyl, Alkenyl, Alkinyl, Aryl, Alkoxy, Alkenoxy, steht,
$R^3$ für Wasserstoff, gegebenenfalls substituiertes Alkyl, Cycloalkyl, Alkenyl, Alkinyl, Aryl, steht,
$R^4$ für Alkyl, Alkoxy, Phenoxy, Alkylthio, Phenylthio, Phenylsulfinyl, Phenylsulfonyl, Benzoyl steht, die gegebenenfalls substituiert sind durch Halogen, Alkoxy, Alkylthio, Halogenalkoxy, Halogenalkylthio
oder Benzimidazolen der Formel II

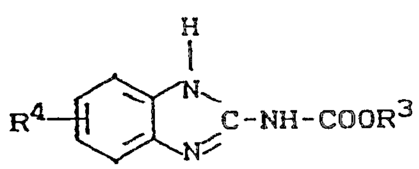

(II)

in welcher
$R^3$ und $R^4$ die bei den Verbindungen der Formel I angegebene Bedeutung haben
und Tetrahydropyrimidinen der Formel III

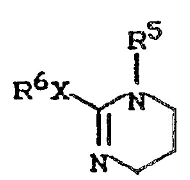

(III)

in welcher
$R^5$ für Wasserstoff oder Alkyl steht,
$R^6$ für gegebenenfalls substituiertes Phenyl oder Thienyl steht,
X für $-(CH_2)_{2-3}-$, $-CH=CH-$ steht,
und gegebenenfalls weiteren Wirkstoffen zur Herstellung von anthelmintischen Mitteln für nichthumane Säugetiere.

5. Mittel gemäß Ansprüchen 1 und 2, dadurch gekennzeichnet, daß als Phenylguanidin Febantel und als Tetrahydropyridin Pyrantel eingesetzt wird.

6. Mittel gemäß Ansprüchen 1 und 2, dadurch gekennzeichnet, daß als Phenylguanidin Febantel, als Tetrahydropyridin Pyrantel sowie als weiterer Wirkstoff Praziquantel eingesetzt wird.

7. Verwendung von Mischungen gemäß Ansprüchen 3 und 4, dadurch gekennzeichnet, daß als Phenylguanidin Febantel oder Netobimin, als Benzimidazol Albendazol, Oxibendazol, Fenbendazol, Flubendazol, Parbendazol, Luxabendazol, oder Oxfendazol und als Tetrahydropyridin Pyrantel, Morantel oder Öxantel und gegebenenfalls weitere Wirkstoffe eingesetzt werden.

8. Verwendung von Mischungen gemäß Ansprüchen 3 und 4, dadurch gekennzeichnet, daß als Phenylguanidin Febantel und als Tetrahydropyridin Pyrantel eingesetzt wird.

9. Verwendung von Mischungen gemäß Ansprüchen 3 und 4, dadurch gekennzeichnet, daß als Phenylguanidin Febantel, als Tetrahydropyridin Pyrantel und als weiterer Wirkstoff Praziquantel eingesetzt wird.

**Patentansprüche für den Vertragsstaat ES :**

1. Verfahren zur Herstellung anthelminthisch wirksamer Mittel, gekennzeichnet durch die Verwendung einer Kombination von Phenylguanidinen der Formel I

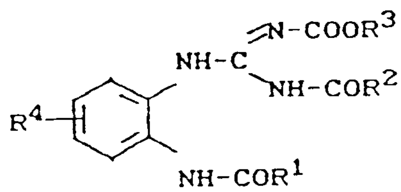

(I)

in welcher
$R^1$ für Wasserstoff, gegebenenfalls substituiertes Alkyl, Cycloalkyl, Alkoxy, Aryl oder Amino steht,
$R^2$ für Wasserstoff, gegebenenfalls substituiertes Alkyl, Cycloalkyl, Alkenyl, Alkinyl, Aryl, Alkoxy, Alkenoxy, steht,
$R^3$ für Wasserstoff, gegebenenfalls substituiertes Alkyl, Cycloalkyl, Alkenyl, Alkinyl, Aryl, Halogenalkoxy, Halogenalkylthio, steht,
$R^4$ für Alkyl, Alkoxy, Phenoxy, Alkylthio, Phenylthio, Phenylsulfinyl, Phenylsulfonyl, Benzoyl steht, die gegebenenfalls substituiert sind durch Halogen, Alkoxy, Alkylthio, Halogenalkoxy, Halogenalkylthio,
oder Benzimidazolen der Formel II

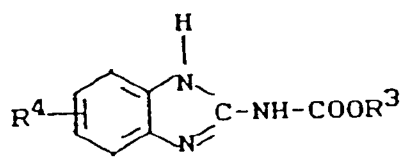

(II)

in welcher
$R^3$ und $R^4$ die bei den Verbindungen der Formel I angegebene Bedeutung haben
zusammen mit Tetrahydropyrimidinen der Formel III

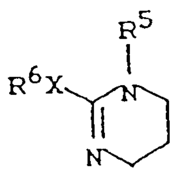

(III)

in welcher
$R^5$ für Wasserstoff oder Alkyl steht,
$R^6$ für gegebenenfalls substituiertes Phenyl oder Thienyl steht,
X für $-(CH_2)_{2-3}-$, $-CH=CH-$ steht

sowie gegebenenfalls weiteren Wirkstoffen, ausgenommen die Mischung zwischen Mebendazol und Pyrantel bzw. Pyrantel-Pamoat.

2. Verfahren gemäß Anspruch 1 zur Herstellung von Mitteln zur Bekämpfung von Hakenwürmern und Spulwürmern.

3. Verwendung von Mischungen aus Phenylguanidinen der Formel I

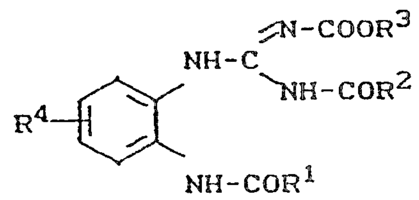

(I)

in welcher
$R^1$ für Wasserstoff, gegebenenfalls substituiertes Alkyl, Cycloalkyl, Alkoky, Aryl oder Amino steht,
$R^2$ für Wasserstoff, gegebenenfalls substituiertes Alkyl, Cycloalkyl, Alkenyl, Alkinyl, Aryl, Alkoxy, Alkenoxy, steht,
$R^3$ für Wasserstoff, gegebenenfalls substituiertes Alkyl, Cycloalkyl, Alkenyl, Alkinyl, Aryl, steht,
$R^4$ für Alkyl, Alkoxy, Phenoxy, Alkylthio, Phenylthio, Phenylsulfinyl, Phenylsulfonyl, Benzoyl steht, die gegebenenfalls substituiert sind durch Halogen, Alkoxy, Alkylthio, Halogenalkoxy, Halogenalkylthio,
oder Benzimidazolen der Formel II

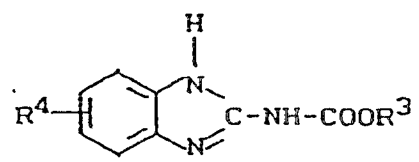

(II)

in welcher
$R^3$ und $R^4$ die bei den Verbindungen der Formel I angegebene Bedeutung haben
mit Tetrahydropyrimidinen der Formel III

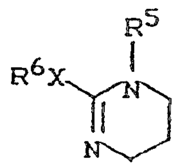

(III)

in welcher
$R^5$ für Wasserstoff oder Alkyl steht,
$R^6$ für gegebenenfalls substituiertes Phenyl oder Thienyl steht,
X für $-(CH_2)_{2-3}-$, $-CH=CH-$ steht,
und gegebenenfalls weiteren Wirkstoffen ausgenommen die Mischung zwischen Mebendazol und Pyrantel bzw. Pyrantel-Pamoat zur Herstellung von anthelmintischen Mitteln.

4. Verwendung von Phenylguanidinen der Formel I

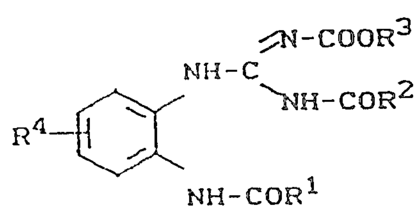

(I)

in welcher
$R^1$ für Wasserstoff, gegebenenfalls substituiertes Alkyl, Cycloalkyl, Alkoxy, Aryl oder Amino steht,
$R^2$ für Wasserstoff, gegebenenfalls substituiertes Alkyl, Cycloalkyl, Alkenyl, Alkinyl, Aryl, Alkoxy, Alkenoxy,

steht,
$R^3$ für Wasserstoff, gegebenenfalls substituiertes Alkyl, Cycloalkyl, Alkenyl, Alkinyl, Aryl, steht,
$R^4$ für Alkyl, Alkoxy, Phenoxy, Alkylthio, Phenylthio, Phenylsulfinyl, Phenylsulfonyl, Benzoyl steht, die gegebenenfalls substituiert sind durch Halogen, Alkoxy, Alkylthio, Halogenalkoxy, Halogenalkylthio
oder Benzimidazolen der Formel II

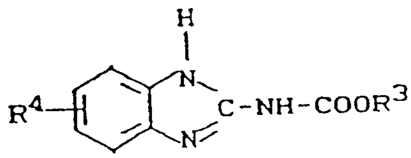

(II)

in welcher

$R^3$ und $R^4$ die bei den Verbindungen der Formel I angegebene Bedeutung haben
und Tetrahydropyrimidinen der Formel III

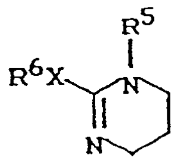

(III)

in welcher $R^5$ für Wasserstoff oder Alkyl steht,
$R^6$ für gegebenenfalls substituiertes Phenyl oder Thienyl steht,
X für $-(CH_2)_{2-3}-$, $-CH=CH-$ steht,
und gegebenenfalls weiteren Wirkstoffen zur Herstellung von anthelmintischen Mitteln für nichthumane Säugetiere.

5. Verfahren gemäß Ansprüchen 1 und 2, dadurch gekennzeichnet, daß als Phenylguanidin Febantel und als Tetrahydropyridin Pyrantel eingesetzt wird.

6. Verfahren gemäß Ansprüchen 1 und 2, dadurch gekennzeichnet, daß als Phenylguanidin Febantel, als Tetrahydropyridin Pyrantel sowie als weiterer Wirkstoff Praziquantel eingesetzt wird.

7. Verwendung von Mischungen gemäß Ansprüchen 3 und 4, dadurch gekennzeichnet, daß als Phenylguanidin Febantel oder Netobimin, als Benzimidazol Albendazol, Oxibendazol, Fenbendazol, Flubendazol, Perbendazol, Luxabendazol oder Oxfendazol und als Tetrahydropyridin Pyrantel, Morantel oder Oxantel und gegebenenfalls weitere Wirkstoffe eingesetzt werden.

8. Verwendung von Mischungen gemäß Ansprüchen 3 und 4, dadurch gekennzeichnet, daß als Phenylguanidin Febantel und als Tetrahydropyridin Pyrantel eingesetzt wird.

9. Verwendung von Mischungen gemäß Ansprüchen 3 und 4, dadurch gekennzeichnet, daß als Phenylguanidin Febantel, als Tetrahydropyridin Pyrantel und als weiterer Wirkstoff Praziquantel eingesetzt wird.

## Claims

### Claims for the Contracting States BE, DE, FR, GB, IT, NL, SE

1. Anthelmintically active agents, characterised in that they contain a combination of phenylguanidines of the formula I

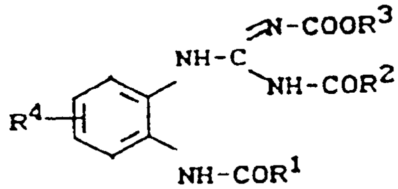

(I)

in which

$R^1$ represents hydrogen, optionally substituted alkyl, cycloalkyl, alkoxy, aryl or amino,

$R^2$ represents hydrogen, optionally substituted alkyl, cycloalkyl, alkenyl, alkinyl, aryl, alkoxy or alkenoxy,

$R^3$ represents hydrogen, optionally substituted alkyl, cycloalkyl, alkenyl, alkinyl, aryl, halogenoalkoxy or halogenoalkylthio,

$R^4$ represents alkyl, alkoxy, phenoxy, alkylthio, phenylthio, phenylsulphinyl, phenylsulphonyl or benzoyl which are in each case optionally substituted by halogen, alkoxy, alkylthio, halogenoalkoxy or halogenoalkylthio,

or benzimidazoles of the formula II

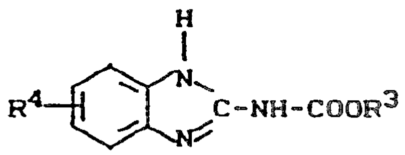

(II)

in which

$R^3$ and $R^4$ have the meaning specified in the case of the compounds of the formula I,

together with tetrahydropyrimidines of the formula III

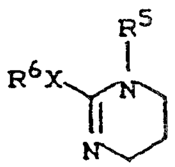

(III)

in which

$R^5$ represents hydrogen or alkyl,

$R^6$ represents optionally substituted phenyl or thienyl, and

X represents $-(CH_2)_{2-3}-$ or $-CH=CH-$ and also, if appropriate, further active compounds, except for the mixture of mebendazole and pyrantel or pyrantel pamoate.

2. Agents according to Claim 1 for combating hookworms and eel worms.

3. Use of mixtures of phenylguanidines of the formula I

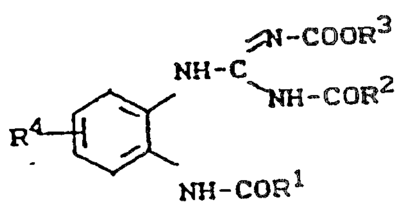

(I)

in which

$R^1$ represents hydrogen, optionally substituted alkyl, cycloalkyl, alkoxy, aryl or amino,

$R^2$ represents hydrogen, optionally substituted alkyl, cycloalkyl, alkenyl, alkinyl, aryl, alkoxy or alkenoxy,

$R^3$ represents hydrogen, optionally substituted alkyl, cycloalkyl, alkenyl, alkinyl or aryl,

$R^4$ represents alkyl, alkoxy, phenoxy, alkylthio, phenylthio, phenylsulphinyl, phenylsulphonyl or benzoyl which are in each case optionally substituted by halogen, alkoxy, alkylthio, halogenoalkoxy or halogenoalkylthio,

or benzimidazoles of the formula II

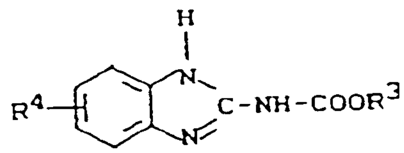

(II)

in which

$R^3$ and $R^4$ have the meaning specified in the case of the compounds of the formula I, with tetrahydropyrimidines of the formula III

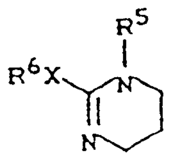

(III)

in which

$R^5$ represents hydrogen or alkyl,

$R^6$ represents optionally substituted phenyl or thienyl, and

X represents $-(CH_2)_{2-3}-$ or $-CH=CH-$, and, if appropriate, further active compounds, except for the mixture of mebendazole and pyrantel or pyrantel pamoate, for the preparation of anthelmintic agents.

4. Use of phenylguanidines of the formula I

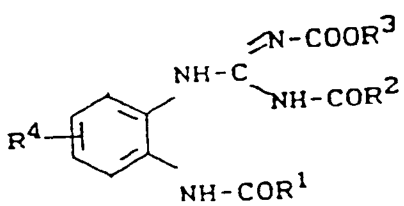

(I)

in which

$R^1$ represents hydrogen, optionally substituted alkyl, cycloalkyl, alkoxy, aryl or amino,

$R^2$ represents hydrogen, optionally substituted alkyl, cycloalkyl, alkenyl, alkinyl, aryl, alkoxy or alkenoxy,

$R^3$ represents hydrogen, optionally substituted alkyl, cycloalkyl, alkenyl, alkinyl or aryl,

$R^4$ represents alkyl, alkoxy, phenoxy, alkylthio, phenylthio, phenylsulphinyl, phenylsulphonyl or benzoyl which are in each case optionally substituted by halogen, alkoxy, alkylthio, halogenoalkoxy or halogenoalkylthio,

or benzimidazoles of the formula II

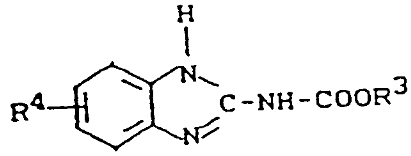

(II)

in which

$R^3$ and $R^4$ have the meaning specified in the case of compounds of the formula I,

and tetrahydropyrimidines of the formula III

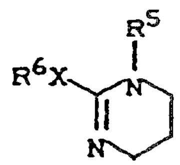

(III)

in which

$R^5$ represents hydrogen or alkyl,

$R^6$ represents optionally substituted phenyl or thienyl, and

X represents $-(CH_2)_{2-3}-$ or -CH=CH-, and, if appropriate further active compounds for the preparation of anthelmintic agents for mammals apart from humans.

5. Agents according to Claims 1 and 2, characterised in that the phenylguanidine employed is febantel and the tetrahydropyridine employed is pyrantel.

6. Agents according to Claims 1 and 2, characterised in that the phenylguanidine employed is febantel, the tetrahydropyridine employed is pyrantel and the further active compound employed is praziquantel.

7. Use of mixtures according to Claims 3 and 4, characterised in that the phenylguanidine employed is febantel or netobimine ; the benzimidazole employed is albendazole, oxibendazole, fenbendazole, flubendazole, parbendazole, luxabendazole or oxfendazole and the tetrahydropyridine employed is pyrantel, morantel or oxantel and if appropriate, other active compounds are employed.

8. Use of mixtures according to Claims 3 and 4, characterised in that the phenylguanidine employed is febantel and the tetrahydropyridine employed is pyrantel.

9. Use of mixtures according to Claims 3 and 4, characterised in that the phenylguanidine employed is febantel, the tetrahydropyridine employed is pyrantel and the further active compound employed is praziquantel.

**Claims for the Contracting States : ES**

1. Process for the preparation of anthelmintically active agents, characterised by the use of a combination of phenylguanidines of the formula I

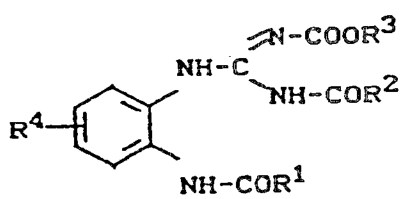

(I)

in which

$R^2$ represents hydrogen, optionally substituted alkyl, cycloalkyl, alkoxy, aryl or amino,

$R^2$ represents hydrogen, optionally substituted alkyl, cycloalkyl, alkenyl, alkinyl, aryl, alkoxy or alkenoxy,

$R^3$ represents hydrogen, optionally substituted alkyl, cycloalkyl, alkenyl, alkinyl, aryl, halogenoalkoxy or halogenoalkylthio,

$R^4$ represents alkyl, alkoxy, phenoxy, alkylthio, phenylthio, phenylsulphinyl, phenylsulphonyl or benzoyl which are in each case optionally substituted by halogen, alkoxy, alkylthio, halogenoalkoxy or halogenoalkylthio,

or benzimidazoles of the formula II

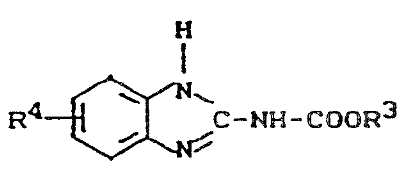

(II)

in which

$R^3$ and $R^4$ have the meaning specified in the case of the compounds of the formula I,

together with tetrahydropyrimidines of the formula III

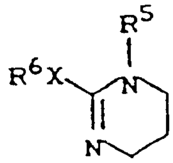

(III)

in which

$R^5$ represents hydrogen or alkyl,

$R^6$ represents optionally substituted phenyl or thienyl, and

X represents $-(CH_2)_{2-3}-$ or $-CH=CH-$ and also, if appropriate, further active compounds, except for the mixture of mebendazole and pyrantel or pyrantel pamoate.

2. Process according to Claim 1 for the preparation of agents for combating hookworms and eelworms.

3. Use of mixtures of phenylguanidines of the formula I

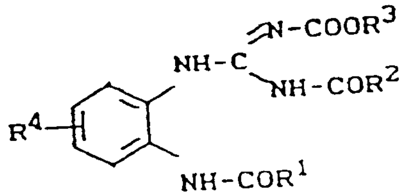

(I)

in which

$R^1$ represents hydrogen, optionally substituted alkyl, cycloalkyl, alkoxy, aryl or amino,

$R^2$ represents hydrogen, optionally substituted alkyl, cycloalkyl, alkenyl, alkinyl, aryl, alkoxy or alkenoxy,

$R^3$ represents hydrogen, optionally substituted alkyl, cycloalkyl, alkenyl, alkinyl or aryl,

$R^4$ represents alkyl, alkoxy, phenoxy, alkylthio, phenylthio, phenylsulphinyl, phenylsulphonyl or benzoyl which are in each case optionally substituted by halogen, alkoxy, alkylthio, halogenoalkoxy or halogenoalkylthio,

or benzimidazoles of the formula II

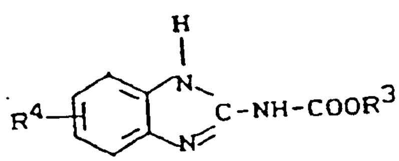

(II)

in which

$R^3$ and $R^4$ have the meaning specified in the case of the compounds of the formula I, with tetrahydropyrimidines of the formula III

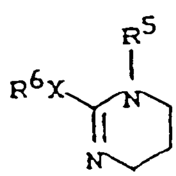

(III)

in which

$R^5$ represents hydrogen or alkyl,

$R^6$ represents optionally substituted phenyl or thienyl, and

X represents $-(CH_2)_{2-3}-$ or $-CH=CH-$, and, if appropriate, further active compounds, except for the mixture of mebendazole and pyrantel or pyrantel pamoate, for the preparation of anthelmintic agents.

4. Use of phenylguanidines of the formula I

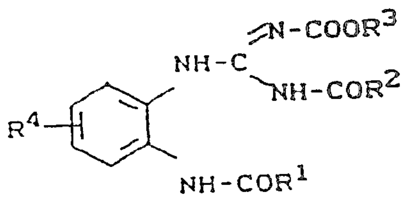

(I)

in which

$R^1$ represents hydrogen, optionally substituted alkyl, cycloalkyl, alkoxy, aryl or amino,

$R^2$ represents hydrogen, optionally substituted alkyl, cycloalkyl, alkenyl, alkinyl, aryl, alkoxy or alkenoxy,

$R^3$ represents hydrogen, optionally substituted alkyl, cycloalkyl, alkenyl, alkinyl or aryl,

$R^4$ represents alkyl, alkoxy, phenoxy, alkylthio, phenylthio, phenylsulphinyl, phenylsulphonyl or benzoyl which are in each case optionally substituted by halogen, alkoxy, alkylthio, halogenoalkoxy or halogenoalkylthio,

or benzimidazoles of the formula II

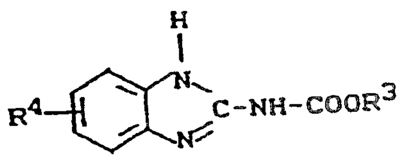

(II)

in which

$R^3$ and $R^4$ have the meaning specified in the case of compounds of the formula I,

and tetrahydropyrimidines of the formula III

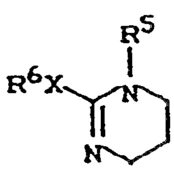

(III)

in which

$R^5$ represents hydrogen or alkyl,

$R^6$ represents optionally substituted phenyl or thienyl, and X represents $-(CH_2)_{2-3}-$ or $-CH=CH-$, and, if appropriate further active compounds for the preparation of anthelmintic agents for mammals apart from humans.

5. Process according to Claims 1 and 2, characterised in that the phenylguanidine employed is febantel and the tetrahydropyridine employed is pyrantel.

6. Process according to Claims 1 and 2, characterised in that the phenylguanidine employed is febantel, the tetrahydropyridine employed is pyrantel and the further active compound employed is praziquantel.

7. Use of mixtures according to Claims 3 and 4, characterised in that the phenylguanidine employed is febantel or netobimine ; the benzimidazole employed is albendazole, oxibendazole, fenbendazole, flubendazole, parbendazole, luxabendazole or oxfendazole and the tetrahydropyridine employed is pyrantel, morantel or oxantel and if appropriate, other active compounds are employed.

8. Use of mixtures according to Claims 3 and 4, characterised in that the phenylguanidine employed is febantel and the tetrahydropyridine employed is pyrantel.

9. Use of mixtures according to Claims 3 and 4, characterised in that the phenylguanidine employed is febantel, the tetrahydropyridine employed is pyrantel and the further active compound employed is praziquantel.

## Revendications

### Revendications pour les Etats Contractants : BE, DE, FR, GB, IT, NL, SE

1. Produit actif antiherminthique, caractérisé en ce qu'il contient une combinaison de phénylguanidines de

formule I

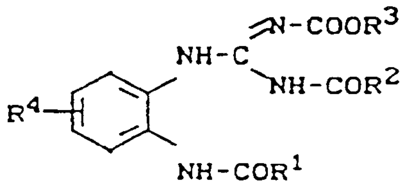

(I)

dans laquelle
$R^1$ représente l'hydrogène, un groupe alkyle, cycloalkyle, alcoxy, aryle ou amino éventuellement substitué,
$R^2$ représente l'hydrogène, un groupe alkyle, cycloalkyle, alcényle, alcynyle, aryle, alcoxy, alcényloxy éventuellement substitué,
$R^3$ représente l'hydrogène, un groupe alkyle, cycloalkyle, alcényle, alcynyle, aryle, halogénoalcoxy, halogénoalkylthio éventuellement substitué,
$R^4$ représente un groupe alkyle, alcoxy, phénoxy, alkylthio, phénylthio, phénylsulfinyle, phénylsulfonyle, benzoyle, ces groupes étant éventuellement substitués par des halogènes, des groupes alcoxy, alkylthio, halogénoalcoxy, halogénoalkylthio,
ou de benzimidazoles de formule II

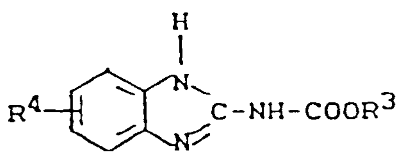

(II)

dans laquelle $R^3$ et $R^4$ ont les significations indiquées en référence aux composés de formule I,
avec des tétrahydropyridines de formule III

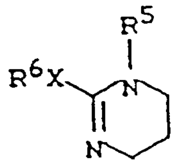

(III)

dans laquelle
$R^5$ représente l'hydrogène ou un groupe alkyle,
$R^6$ représente un groupe phényle ou thiényle éventuellement substitué,
X représente $-(CH_2)_{2\text{ à }3}-$, $-CH=CH-$
et le cas échéant d'autres substances actives, à l'exception du mélange entre le Mebendazol et le Pyrantel ou le pamoate de Pyrantel.

2. Produit selon la revendication 1, pour la lutte contre les ankylostomes et les ascarides.

3. Utilisation des mélanges de phénylguanidines de formule I

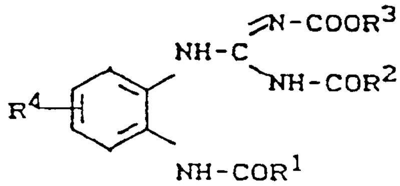

(I)

dans laquelle
$R^1$ représente l'hydrogène, un groupe alkyle, cycloalkyle, alcoxy, aryle ou amino éventuellement substitué,
$R^2$ représente l'hydrogène, un groupe alkyle, cycloalkyle, alcynyle, alcényle, aryle, alcoxy, alcényloxy éven-

tuellement substitué,
$R^3$ représente l'hydrogène, un groupe alkyle, cycloalkyle, alcényle, alcynyle, aryle éventuellement substitué,
$R^4$ représente un groupe alkyle, alcoxy, phénoxy, alkylthio, phénylthio, phénylsulfinyle, phénylsulfonyle, benzoyle, tous ces groupes étant éventuellement substitués par des halogènes, des groupes alcoxy, alkylthio, halogénoalcoxy, halogénoalkylthio,
ou de benzimidazoles de formule II

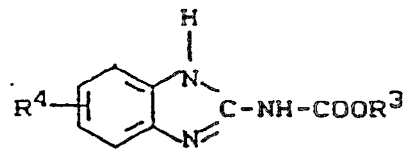

(II)

dans laquelle $R^3$ et $R^4$ ont les significations indiquées en référence aux composés de formule I,
avec des tétrahydropyridines de formule III

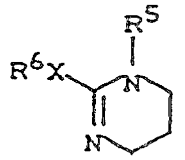

(III)

dans laquelle
$R^5$ représente l'hydrogène ou un groupe alkyle,
$R^6$ représente un groupe phényle ou thiényle éventuellement substitué,
X représente $-(CH_2)_{2\ à\ 3}-$, $-CH=CH-$,
et le cas échéant d'autres substances actives, à l'exception du mélange entre le Mebendazol et le Pyrantel ou le pamoate de Pyrantel, pour la préparation de produits antihelminthiques.

4. Utilisation de phénylguanidines de formule I

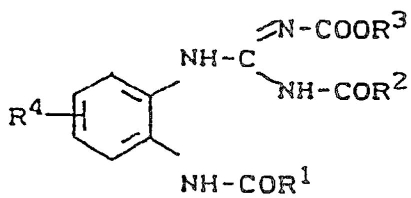

(I)

dans laquelle
$R^1$ représente l'hydrogène, un groupe alkyle, cycloalkyle, alcoxy, aryle ou amino éventuellement substitué,
$R^2$ représente l'hydrogène, un groupe alkyle, cycloalkyle, alcényle, alcynyle, aryle, alcoxy, alcényloxy éventuellement substitué,
$R^3$ représente l'hydrogène un groupe alkyle, cycloalkyle, alcényle, alcynyle, aryle éventuellement substitué,
$R^4$ représente un groupe alkyle, alcoxy, phénoxy, alkylthio, phénylthio, phénylsulfinyle, phénylsulfonyle, benzoyle, tous ces groupes éventuellement substitués par des halogènes, des groupes alcoxy, alkylthio, halogénoalcoxy, halogénoalkylthio,
ou de benzimidazoles de formule II

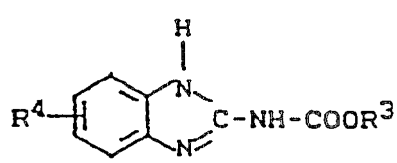

(II)

dans laquellle $R^3$ et $R^4$ ont les significations indiquées en référence aux composés de formule I,
et de tétrahydropyridines de formule III

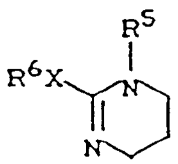

(III)

dans laquelle
$R^5$ représente l'hydrogène ou un groupe alkyle,
$R^6$ représente un groupe phényle ou thiényle éventuellement substitué,
X représente $-(CH_2)_{2\ à\ 3}-$, $-CH=CH-$,
et le cas échéant d'autres substances actives,
pour la préparation de produits antihelminthiques pour des mammifères autres que l'homme.

5. Produit selon les revendications 1 et 2, caractérisé en ce qu'il contient en tant que phénylguanidine le Febantel et en tant que tétrahydropyridine le Pyrantel.

6. Produit selon les revendications 1 et 2, caractérisé en ce qu'il contient en tant que phénylguanidine le Febantel, en tant que tétrahydropyridine le Pyrantel et en tant qu'autre substance active le Praziquantel.

7. Utilisation des mélanges selon les revendications 3 et 4, caractérisée en ce que l'on utilise en tant que phénylguanidine le Febantel ou le Netobimin, en tant que benzimidazole l'Albendazol, l'Oxibendazol, le Fenbendazol, le Flubendazol, le Parbendazol, le luxabendazol ou l'Oxfendazol et'en tant que tétrahydropyridine le Pyrantel, le Morantel ou l'Oxantel avec, le cas échéant, d'autres substances actives.

8. Utilisation des mélanges selon les revendications 3 et 4, caractérisée en ce que l'on utilise en tant que phénylguanidine le Febantel et en tant que tétrahydropyridine le Pyrantel.

9. Utilisation des mélanges selon les revendications 3 et 4, caractérisée en ce que l'on utilise en tant que phénylguanidine le Febantel, en tant que tétrahydropyridine le Pyrantel et en tant qu'autre substance active le Praziquantel.

**Revendications pour l'Etat Contractant : ES**

1. Procédé de préparation de produits actifs antihelminthiques, caractérisé en ce que l'on utilise une combinaison de phénylguanidines de formule I

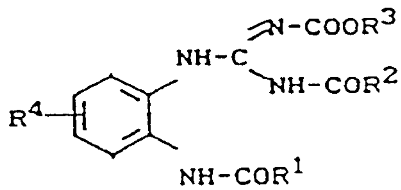

(I)

dans laquelle
$R^1$ représente l'hydrogène un groupe alkyle, cycloalkyle, alcoxy, aryle ou amino éventuellement substitué,
$R^2$ représente l'hydrogène, un groupe alkyle, cycloalkyle, alcényle, alcynyle, aryle, alcoxy, alcényloxy éventuellement substitué,
$R^3$ représente l'hydrogène, un groupe alkyle, cycloalkyle, alcényle, alcynyle, aryte, haloénoalcoxy, halogénoalkylthio éventuellement substitué,
$R^4$ représente un groupe alkyle, alcoxy, phénoxy, akylthio, phénylthio, phénylsulfinyle, phénylsulfonyle, benzoyle, tous ces groupes éventuellement substitués par des halogènes, des groupes alcoxy, alkylthio, halogénoalcoxy, halogénoalkylthio,
ou de benzimidazoles de formule II

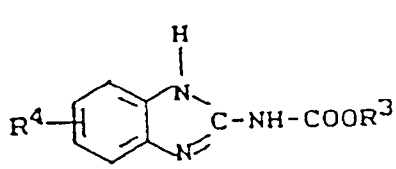

(II)

dans laquelle $R^3$ et $R^4$ ont les significations indiquées en référence aux composés de formule I,

avec des tétrahydropyridines de formule III

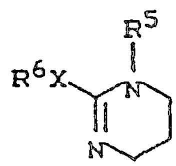

(III)

dans laquelle
$R^5$ représente l'hydrogène ou un groupe alkyle,
$R^6$ représente un groupe phényle ou thiényle éventuellement substitué,
X représente $-(CH_2)_{2\text{ à }3}-$, $-CH=CH-$,
et le cas échéant d'autres substances actives, à l'exception du mélange entre le Mebendazol et le Pyrantel ou le pamoate de Pyrantel,

2. Procédé selon la revendication 1, pour la préparation de produits pour la lutte contre les ankylostomes et les ascarides.

3. Utilisation de mélanges de phénylguanidines de formule I

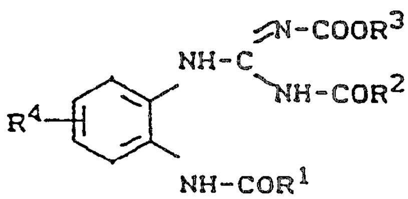

(I)

dans laquelle
$R^1$ représente l'hydrogène un groupe alkyle, cycloalkyle, alcoxy, aryle ou amino éventuellement substitué,
$R^2$ représente l'hydrogène un groupe alkyle, cycloalkyle, alcényle, alcynyle, aryle, alcoxy, alcényloxy éventuellement substitué,
$R^3$ représente l'hydrogène, un groupe alkyle, cycloalkyle, alcényle, alcynyle, aryle éventuellement substitué,
$R^4$ représente un groupe alkyle, alcoxy, phénoxy, alkylthio, phénylthio, phénylsulfinyle, phénylsulfonyLe, benzoyle, tous ces groupes éventuellement substitués par des halogènes, des groupes alcoxy, alkylthio, halogénoalcoxy, halogénoalkylthio,
ou de benzimidazoles de formule II

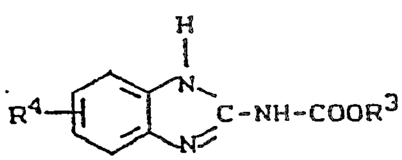

(II)

dans laquelle $R^3$ et $R^4$ ont les significations indiquées en référence aux composés de formule I,
avec des tétrahydropyridines de formule III

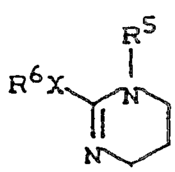

(III)

dans laquelle
$R^5$ représente l'hydrogène ou un groupe alkyle,
$R^6$ représente un groupe phényle ou thiényle éventuellement substitué,
X représente $-(CH_2)_{2\text{ à }3}-$ ou $-CH=CH-$,

et le cas échéant d'autres substances actives, à l'exception du mélange entre le Mebendazol et le Pyrantel ou le pamoate de Pyrantel,
pour la préparation de produits antihelminthiques.

4. Utilisation des phénylguanidines de formule I

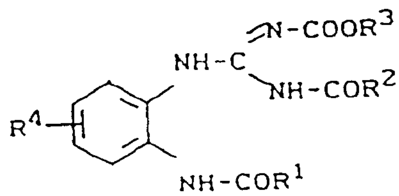

(I)

dans laquelle
$R^1$ représente l'hydrogène, un groupe alkyle, cycloalkyle, alcoxy, aryle ou amino éventuellement substitué,
$R^2$ représente l'hydrogène un groupe alkyle, cycloalkyle, alcényle, alcynyle, aryle, alcoxy, alcényloxy éventuellement substitué,
$R^3$ représente l'hydrogène, un groupe alkyle, cycloalkyle, alcényle, alcynyle, aryle éventuellement substitué,
$R^4$ représente un groupe alkyle, alcoxy, phénoxy, alkylthio, phénylthio, phénylsulfinyle, phénylsulfonyle, benzoyle, tous ces groupes éventuellement substitués par des halogènes, des groupes alcoxy, alkylthio, halogénoalcoxy, halogénoalkylthio,
ou de benzimidazoles de formule II

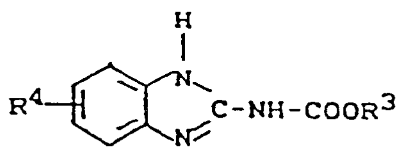

(II)

dans laquelle $R^3$ et $R^4$ ont les significations indiquées en référence aux composé de formule I,
et de tétrahydropyridines de formule III

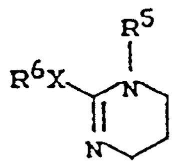

(III)

dans laquelle
$R^5$ représente l'hydrogène ou un groupe alkyle,
$R^6$ représente un groupe phényle ou thiényle éventuellement substitué,
X représente $-(CH_2)_{2\ à\ 3}-$, $-CH=CH-$,
et le cas échéant d'autres substances actives, pour la préparation de produits antihelminthiques pour des mammifères autres que l'homme.

5. Procédé selon les revendications 1 et 2, caractérisé en ce que l'on utilise en tant que phénylguanidine le Febantel et en tant que tétrahydropyridine le Pyrantel.

6. Procédé selon les revendications 1 et 2, caractérisé en ce que l'on utilise en tant que phénylguanidine le Febantel, en tant que tétrahydropyridine le Pyrantel et en tant qu'autre substance active le Praziquantel.

7. Utilisation des mélanges selon les revendications 3 et 4, caractérisée en ce que l'on utilise en tant que phénylguanidine le Febentel ou le Netobimin, en tant que benzimidazole l'Albendazol, l'Oxibendazol, le Fenbendazol, le Flubendazol, le Perbendazol, le Luxabendazol ou l'Oxfendazol et en tant que tétrahydropyridine le Pyrantel, le Morantel ou l'Oxantel avec le cas échéant d'autres substances actives.

8. Utilisation des mélanges selon les revendications 3 et 4, caractérisée en ce que l'on utilise en tant que phénylguanidine le Febantel et en tant que tétrahydropyridine le Pyrantel.

9. Utilisation des mélanges selon les revendications 3 et 4, caractérisée en ce que l'on utilise en tant que phénylguanidine le Febantel, en tant que tétrahydropyridine le Pyrantel et en tant qu'autre substance active le Praziquantel.